# EUROPEAN PATENT APPLICATION

(11) **EP 4 818 329 A1**
(43) Date of publication of application: **07.10.2026**
(21) Application number: 25167944.5
(22) Date of filing: 02.04.2025
(51) Int. Cl.: C12Q 1/6876, C12Q 1/6816

(54) **NUCLEIC ACID NANOPORE WITH CLOSING AND OPENING NUCLEIC ACID**

(71) Applicant: AIT Austrian Institute of Technology GmbH, 1210 Wien (AT)
(72) Inventor: BARISIC, Ivan, 1150 Wien (AT); ANDERSSON, Jakob, 1090 Wien (AT)
(74) Representative: SONN Patentanwälte GmbH & Co KG

(57) **Abstract**

The invention provides a nanopore assembly comprising a nucleic acid nanopore that penetrates a lipid membrane, wherein said lipid membrane is tethered to a solid surface, and with the nucleic acid nanopore comprising a lumen with openings, with a first openings oriented at the solid surface and a second opening oriented away from the surface, wherein at at least one of the openings the nucleic acid nanopore comprises a lid nucleic acid, wherein said lid nucleic acid comprises a hybridizing sequence that is complementary to a sequence of the nucleic acid nanopore located flanking the opening at which it is located, and wherein the solid surface comprises an electrical conductor.

## Description

### Background

Nucleic acids, such as cell-free DNA (cfDNA), have become an important biomarker for detecting a wide range of diseases or conditions, such as cancer diagnostics, prenatal testing, organ transplant monitoring, and infectious disease diagnostics. However, the current methods to detect short nucleic acids are often slow, expensive, laborious, and mostly based on sequencing technologies. Especially in the field of infectious diseases, rapid and cost-effective diagnostic testing is essential because the first 6-12 h are crucial for the treatment of life-threatening bacterial infections. Rapid, label-free, and amplification-independent detection of nucleic acids is desired to improve diagnostic procedures and enable fast therapeutic responses. Currently, this class of biomarkers is mainly detected using sequencing technologies which are neither fast nor cost-effective and require highly trained personnel.

The current gold standard in diagnosing bacterial infections is based on the cultivation of bacteria, subsequent Gram staining, species identification, and antimicrobial susceptibility testing (AST) and typically requires 3-5 days. Another promising approach for sepsis analysis is based on metagenomics. It targets degraded microbial cfDNAs that are circulating in the blood stream. These have the potential to be even more sensitive than cultivation-based approaches (Hassall et al. npj Antimicrob Resist 2; 10.1038/s44259-024-00033-8 (2024); Christians et al., J Clin Microbiol 62(10): 10.1128/jcm.00425-24). Although cultivation-independent, the cfDNA extraction, library preparation, sequencing, and bioinformatic analyses require still long turnaround times, highly specialised laboratories, and are resource-intensive.

Thus, even more rapid, and simple methods to analyse cfDNA are required to harness the full potential of this promising biomarker. It is therefore a goal to provide an improved sensing method to detect DNA analytes, particularly cfDNA.

### Summary of the Invention

The invention relates to a nanopore assembly comprising a nucleic acid nanopore that penetrates a lipid membrane, wherein said lipid membrane is tethered to a solid surface, and with the nucleic acid nanopore comprising a lumen with openings, with a first opening oriented at the solid surface and a second opening oriented away from the surface, wherein at at least one of the openings the nucleic acid nanopore comprises a lid nucleic acid, where-in said lid nucleic acid comprises a hybridizing sequence that is complementary to a sequence of the nucleic acid nanopore located flanking the opening at which it is located, and wherein the solid surface comprises an electrical conductor.

In a further but related aspect the invention provides a method of detecting an analyte that binds to a nucleic acid, comprising providing a nanopore assembly of the invention, wherein the lid nucleic acid is specific to binding an analyte of interest, comprising contacting the nanopore assembly with a sample comprising the analyte and detecting an electrical property in case the analyte of interest binds the lid nucleic acid.

Furthermore, the invention provides a method of generating the nanopore assembly of the invention, comprising immobilizing a lipid membrane to a solid surface, wherein the solid surface comprises an electrical conductor and integrating a nucleic acid nanopore into the lipid membrane, wherein the nucleic acid nanopore comprises a lumen with openings, and wherein at at least one of the openings the nucleic acid nanopore comprises a lid nucleic acid, wherein said lid nucleic acid comprises a hybridizing sequence that is complementary to a sequence of the nucleic acid nanopore located flanking the opening at which it is located, and further comprising hydrophobic anchors.

All aspects of the invention are related and any description of one aspect equally reads on the other aspects. E.g**.** as description of the nanopore assembly or any part thereof, in particular the nanopore, reads on a nanopore assembly or part thereof used in the inventive detection method or on a product obtained in method of generating the nanopore assembly or part thereof, such as a suitability to generate that nanopore assembly. A description of the detection method also reads on a suitability of the nanopore assembly for that method. A description of the method of generation also reads on the products, i.e**.** the nanopore assembly of the invention.

### Brief description of the figures

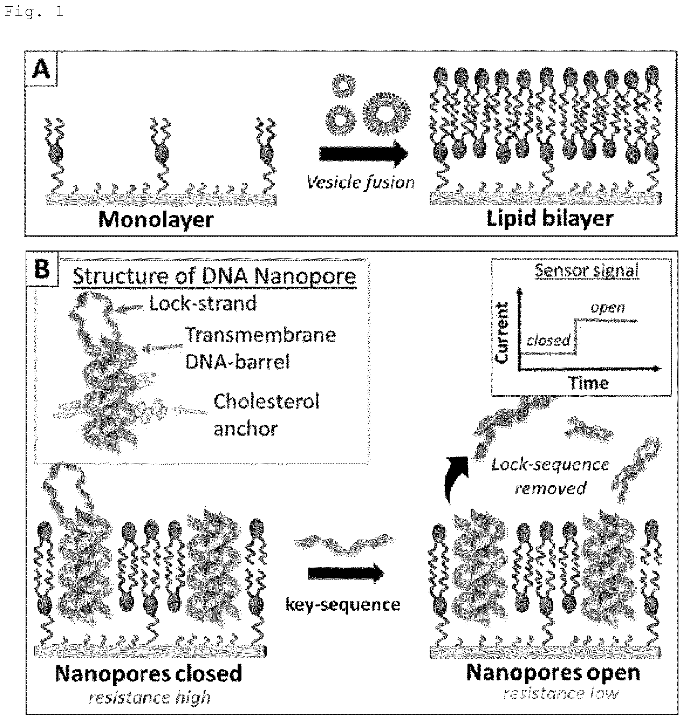

Fig. 1: Production of the nanopore assembly. A) Tethered lipids are bound to a conductive surface. These tethered lipids form a lipid bilayer together with lipids from vesicles via vesicle fusion. B) A DNA nanopore comprising cholesterol anchors integrates into the lipid bilayer. With the addition of a target or "key" sequence the lid nucleic acid or "lock" is removed from a closing position to form an open position. The difference between the closed and open positions can be measured by an altered current as sensor signal.

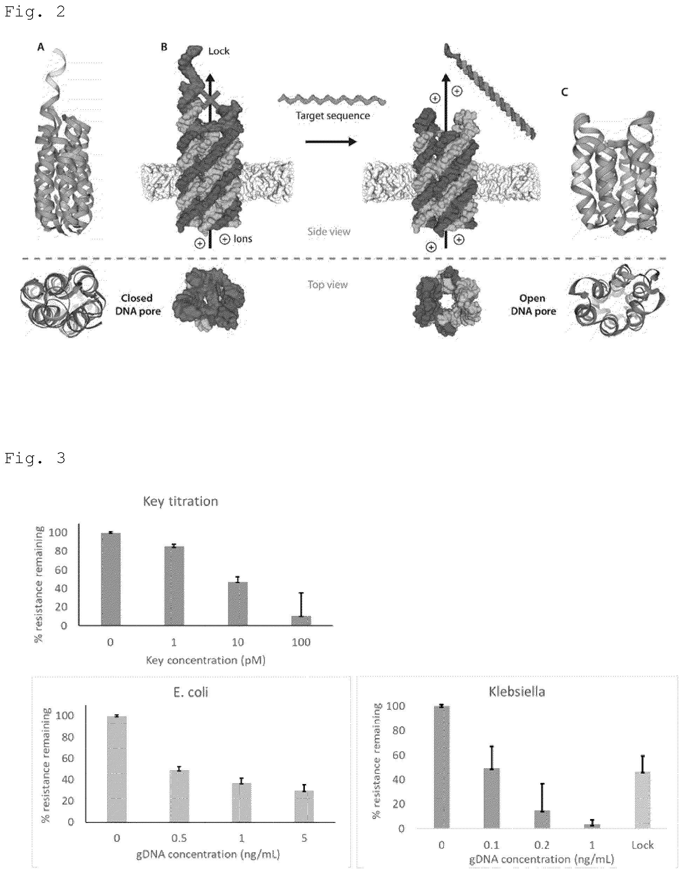

Fig. 2: Figure 1. Structural models of a nanopore in closed and open state, for each depiction: top - side view onto the nanopore and lipid membrane; bottom - top view. A) Structural model of the closed pore predicted with AlphaFold3 (Abramson et al. Nature 630: 493-500 (2024)). B) Atomistic model of the DNA nanopore made in Catana (Kut'ák et al. Nucleic acids research DOI: 10.1093/nar/gkac350 (2022)) and its integration in a lipid bilayer. A lid nucleic acid (in red) acts as a lock and closes the pore. As shown in the top view (bottom), the lid nucleic acid is across the opening of the pore. Upon addition of a target sequence (green), the lock strand is removed from the pore via strand displacement. This process removes the lid and enables the movement of ions through the pore. C) Structural model of the open nanopore predicted with AlphaFold3.
Fig. 3: A) Graph illustrating the sensor response in terms of reductions in membrane resistance in response to different concentrations of a target oligonucleotide (bla_{OXA-48}) and non-target sequences.

### Detailed Description

The invention relates to a nanopore assembly, i.e. a nanopore assembled for the inventive utilities, in particular DNA sensing methods. The nanopore assembly comprises a nucleic acid nanopore, a lipid membrane and a solid surface. The lipid membrane is tethered to the solid surface. The nucleic acid nanopore (also referred to just as "nanopore") is embedded in the lipid membrane. In particular, the nucleic acid nanopore penetrates the lipid membrane, i.e. it provides a pore through the lipid membrane via the nanopore's lumen. The lipid membrane has two sides, one facing the solid surface and one facing away from the solid surface. Both sides are connected through the lumen of the nanopore. The nucleic acid nanopore comprises a lumen with openings, with a first openings oriented at the solid surface (at the side of the lipid membrane facing the solid surface) and a second opening oriented away from the surface (at the side of the lipid membrane facing away from the solid surface). At at least one of the openings the nucleic acid nanopore comprises a lid nucleic acid. When the lid nucleic acid is across the opening at which it is located, then the lid nucleic acid hinders flow through the nanopore since it blocks the opening, at least partially. This is referred to as closed state. When the lid nucleic acid is removed from the opening the flow through the nanopore is increased as compared to the closed state. This is referred to as the open state or an unblocked state since the lid nucleic acid is removed from the blocking configuration.

Blocking and unblocking or in other words closing and opening can be mediated by hybridization of a target nucleic acid to the lid nucleic acid. The target nucleic acid can be an analyte of interest that is measured or detected with the inventive nanopore, such as a cfDNA as just one example. Of course, other target nucleic acids can be used. To bind the lid nucleic acid to the nanopore, the lid nucleic acid comprises a hybridizing sequence that is complementary to a sequence of the nucleic acid nanopore located flanking the opening at which the lid nucleic acid is located.

For blocking (closed state) the lid nucleic acid can be located across the opening at which it is located, e.g. across the second opening. The greater the area coverage of the opening by the lid nucleic acid, the more efficient the blockage of the flow through the nanopore will be. For placement across the opening, the lid nucleic acid can be bound to moieties of the nanopore that are on opposing positions in the surrounding of the opening. A preferred nanopore comprises a helix double structure, comprising several double helices. The lid nucleic acid may be bound to two (or more) double helices that are opposite to each other in their location in the nanopore at the opening. For example, in a six helices bundle, the lid nucleic acid may be bound to helices 1 and 4 (or 2 and 5; or 3 and 6) with the helices numbered in sequence following around the opening. Of course, the nanopore may have other quantities of helices and a skilled person can easily select the best binding partner for the lid nucleic acid at the nanopore.

Given that the lid nucleic acid and the nucleic acid nanopore are composed of nucleic acids and easy mode for binding the lid nucleic acid to the nanopore is by nucleic acid hybridization. This binding type is preferred in all embodiments of the invention. Accordingly, the lid nucleic acid may have one or more sequence(s) that is complementary to one or more sequence(s) of the nanopore, preferably the complementary sequence is at least 4 nucleotides in length, more preferred, 5, 6, 7, 8, 9, 10, 11, 12, 13, 14, or more nucleotides in length. Preferably the complementary sequence has a length of 4-8 complementary nucleotides. Optionally and preferred, the complementary sequences of the nanopore for binding the lid nucleic acid are placed at a loop, e.g. within 0, 1, 2, 3, 4, 5, or 6, nucleotides to a loop. Alternatively or in combination thereto, a strand of the nanopore may contain one or preferably two such complementary sequences, e.g. flanking a loop. Preferably, the lid is bound to the nanopore by at least two separate binding regions, preferably by at least two complementary sequences, e.g. for binding across the opening as discussed above. A separate binding region may comprise one or two or more complementary sequences of the nanopore for binding the lid nucleic acid. A binding region may contain a loop as mentioned above. Binding of the lid to the remaining nanopore is also referred to as "docking". It is a reversible binding and during the inventive detecting methods, the lid nucleic acid shall be displaced from one or more of the binding regions of the nanopore by the target analyte, which causes the transition from the closed to the open state of the nanopore.

The term "complementary sequences" refers to the ability of nucleic acids to hybridize by Watson-Crick base pairing to each other. Natural bases pair according to A-T(U) or G-C and in opposite direction (reverse), i.e. a sequence in 5'-3' binds to a complementary sequence that is in 3'-5' direction. A sequence that is complementary to another sequence thus comprises the complementary nucleotides in reversed order.

Possible nucleic acids are DNA, RNA or artificially modified nucleotides, such as peptide nucleic acids, or mixtures thereof. Preferably the nucleic acid nanopore comprises DNA nucleotides, especially preferred in a large amount, e.g. nucleic acid nanopore comprises nucleotides of which 80% to 100% are DNA nucleotides. The conformation of the nucleic acids is preferably in a B-DNA conformation, optionally (and preferred) with hairpin loops. The B-DNA conformation allows an easy folding into the double helix structure for complementary nucleotides and sequences. Hairpin loops can be used in linked double helix structures to link neighboring double helices.

Designing nucleic acid nanopores is nowadays an easy task, that can be accomplished with base pairing prediction software or folding predictions, e.g. on a computer. An example of such designs is given by 'DNA origami'. Membrane spanning nucleic acid nanopores are typically comprised of a structural core of a plurality of interlinked nucleic acid duplexes that enclose a hollow channel, open at both ends. The technique of DNA origami can be used to create a nucleic acid nanopore that penetrates and spans a lipid membrane (Langecker et al., Science 338(6109): 932-936 (2012), Burns et al., Nature nanotechnology 11: 152-156 (2016), each of which is incorporated by reference in its entirety). In this way the nucleic acid nanopore mimics the configuration of analogous protein nanopores. Optionally, a barrel-shaped cap adheres to one side of the membrane and extends outwardly to form a funnel.

In a preferred embodiment, the nanopore of the invention comprises one or more polynucleotide strands that provide a functional scaffold component, wherein the polynucleotide strands comprised within the scaffold component include a polynucleotide backbone; and a plurality of polynucleotide strands that provide a plurality of functional staple components. The scaffold strand(s) cooperate with and hybridize to the plurality of staple polynucleotide strands - e.g. via appropriate Watson-Crick base pairing hybridization - in order to form a three-dimensional configuration of the nanopore. Accordingly, the nanopores of the present invention can be assembled via the 'scaffold-and-staple' approach. In this approach to nucleic acid nanostructure, in particular, DNA nanostructures, one or more nucleic acids are utilized as a building material in order to make nanoscale three dimensional shapes. Assembly of these nanostructures from a plurality of un-hybridized linear molecules is typically referred to as 'DNA origami', although the technique is equally applicable to other nucleic acids.

The DNA origami process generally involves the folding of the one or more elongate, 'scaffold' DNA strands into a particular shape using a plurality of rationally designed 'staple' DNA strands. The scaffold strand can have any sufficiently non-repetitive sequence. The sequences of the staple strands are designed such that they hybridize to portions of the scaffold strands and, in doing so, these two components cooperate force the scaffold strands to assume a particular structural configuration. Methods useful in the making of DNA origami structures can be found, for example, in Rothemund, P.W., Nature 440:297-302 (2006); Douglas et al, Nature 459:414- 418 (2009); Douglas et al., Nucleic Acids Research 37(15): 5001-5006 (2009); Dietz et al, Science 325:725-730 (2009); and US 2007/0117109, US 2008/0287668, US 2010/0069621, US 2010/0216978, WO 2018/011603 A1, WO 2020/025974 A1, WO 2022/064202 A1, WO 2022/263670 A1, and WO 2022/263669 A1, each of which is incorporated by reference in its entirety. Staple sequence design can be facilitated using, for example, CaDNAno software, available at http://www.cadnano.org. To create a nanopore, the design often includes a central cavity or channel surrounded by DNA helices. The staple strands are designed such that they leave an open space in the middle, which forms the pore. The size and shape of the pore can be precisely controlled by adjusting the design of the staples.

The classical scaffold and stable structure is not a requirement. It is also possible to design the pore based on concatenated nucleic acid strands, for example linked helices. The design process can optionally be aided by using DNA folding prediction software, e.g. AlphaFold3. The links between the helices can be at the termini of the helices, to bind two neighboring helices together. This works especially well with short helices, such as helices with lengths of 25 nt (nt ... nucleotides) to 500 nt in length, especially preferred 30 nt to 250 nt in length. An example of such a nucleic acid nanopore comprises the sequences of SEQ ID NOs. 1-6. These sequences may be interrupted by inserts, e.g. for binding the lid nucleic acid, e.g. SEQ ID NO: 7, which is based on SEQE ID NO: 2 (and can replace it) and further contains such binding sequences and e.g. SEQ ID NO: 8 which is based on SEQE ID NO: 5 (and can replace it) and further contains such binding sequences. Each of SEQ ID NOs 1-6 (and optionally SEQ ID NO: 7 instead of SEQ ID NO: 2 and/or SEQ ID NO: 8 instead of SEQ ID NO: 5) is a part of two DNA double helices with neighboring sequences; these double helices are connected at each helix end by a hairpin-loop at each end of the helices and within the SEQ ID NO. 1-6 sequences (or SEQ ID NO: 7-8 sequences). In addition, SEQ ID NOs 7 and 8 contain dock sequences that bind to the lid nucleic acid (SEQ ID NO: 9 in the examples). The dock sequences preferably do not bind to another nucleic acid strand of the nanopore apart from the lid nucleic acid, e.g. by sterically separation of non-complementary within a sequence of at least 6, preferably at least 4, nucleotides to another strand in their vicinity (based on the predicted folding structure).

Each of SEQ ID NOs. 1-6 (and/or SEQ ID NOs 7-8) can also be modified; possible sequences of one or more helices of an inventive nanopore may comprise 75% or more sequence identity to any one of SEQ ID NO: 1-6 (and/or SEQ ID NOs 7-8). Any one of SEQ ID NO. 1-6 may further comprise a further sequence, e.g. as in insert within the sequence, for binding the lid nucleic acid. Such an insert is preferably 6 to 100 nucleotides in length, more preferably 8 to 60 nucleotides in length, even more preferred 9 to 40 nucleotides in length. Examples of such modified nucleic acids with inserts are SEQ ID NO: 7 and 8. The insert is preferably at a loop and/or comprises a loop. A loop may for example comprise the sequence TTTT or any other loop sequence known in the art.

Preferably the nucleic acid nanopore comprises at least 3 nucleic acid double helices; the nucleic acid nanopore may comprise 4, 5, 6, 7, 8, 9, 10, 11, 12, 13, 14, 15, 16, 17, 18, 19, 20, 21, 22, 23, 24, 25, 26, 27, 28, 29, 30 or more nucleic acid double helices, especially nucleic acid double helices of at least 20 base pairs. Preferably the nucleic acid nanopore comprises 4 to 25 nucleic acid double helices, even more preferred 5 to 12 nucleic acid double helices, in particular preferred are 6 to 8 nucleic acid double helices, which are an optimal compromise between lumen size, of which a bigger one is beneficial for flow-through, and functional stability and reduced leakiness, for which a smaller size is preferable.

Preferably the nucleic acid nanopore comprises nucleic acid double helices with a length of 40 nucleotides to 500 nucleotides, preferably 45 nucleotides to 200 nucleotides. Also, preferred, the nucleic acid nanopore comprises nucleic acid double helices with a length of 20 base pairs to 250 base pairs, preferably 22 base pairs to 100 base pairs. Preferably each of the nanopore's double helices has such lengths. The length shall be sufficient to provide a nanopore that reaches through the entire width of the lipid membrane. Some overshooting length may be beneficial to establish stable openings of the nanopore outside of the lipid membrane, e.g. by providing a structure outside of the lipid membrane that is solvated by the medium outside of the lipid membrane, usually an aqueous medium. The nanopore can be larger but preferably its size, especially its length protruding into the medium, is kept small to reduce leakiness.

Preferably, the nucleic acid nanopore has a length of 4 nm to 30 nm. Length shall be the in the dimension traversing the lipid membrane, in particular perpendicular to the plane of the lipid membranes. Given that the lipid membrane is parallel to the solid surface, this dimension is also perpendicular to the solid surface. The length can be determined by structure determination software given the predicted folding pattern of the nucleic acid. Preferred lengths are sufficient to traverse the lipid membrane, i.e**.** a length greater than the thickness of the lipid membrane. Preferably the nucleic acid nanopore is not overly long to reduce leakiness. A preferred length is thus 5 nm to 25 nm, even more preferred 6 nm to 20 nm especially preferred 7 nm to 15 nm.

The nanopore has a lumen between two openings, openings on each side of the lipid membrane. Thus, the lumen connects both outsides of the lipid membrane or in other words, it connects the medium on each side of the lipid membrane. Medium contents, such as ions can flow through the openings and lumen in the open state. Such a flow-through is blocked or hindered in the closed state of the nanopore. For efficient flow-through, preferably the lumen has a diameter of at least 1.2 nm along its length from first opening to second opening. Preferably that diameter is 1.2 nm to 5 nm, more preferred 1.4 nm to 4 nm, e.g. 1.6 nm to 3 nm. Ions are a preferred medium content to pass through as they can be easily measured by electrical measurements, e.g. by detecting an electrical property, such as impedance, current, voltage or resistance. The diameter of the lumen can be determined similarly as the length of the nanopore, e.g. by imaging a folded structure of the nanopore, e.g. as modelled by AlphaFold3. The diameter shall be in the open state of the nanopore, i.e. without the lid nucleic acid. In case of a non-circular cross section of the lumen (cross section in the plane of the lipid membrane) the diameter shall refer to the smallest dimension in the cross section. Suitably, the nanopore has a consistent minimum diameter for its entire length from first to second opening.

The lid nucleic acid is specific to binding an analyte of interest (the analyte or target). As mentioned above, binding of the analyte to the lid nucleic acid displaces the lid nucleic acid from the position at the opening or across the opening and facilitates the transition from the closed to the open state of the nanopore. The lid is thus a removable blockage of the nanopore. Binding of the analyte to the lid nucleic acid is preferably by nucleic acid base pairing, e.g. the lid comprises a complementary sequence to the sequences of the analyte. In most cases the analyte is a nucleic acid. Other analytes that bind to the lid nucleic acid are also possible, such as antibodies, or other chemical compounds. The lid nucleic acid may be an aptamer that binds the analyte. Especially preferred, the analyte is a nucleic acid, in particular DNA or RNA. Especially preferred is cfDNA. Preferably the lid nucleic acid has a longer sequence that is complementary to the analyte than it has complementary sequence(s) to the other parts of the nanopore. Preferably, the lid nucleic has a sequence that is complementary to the analyte of at least 12 nucleotides, preferably of at least 16 nucleotides, more preferred of at least 20 nucleotides, in length. Example lengths of a sequence that is complementary to the analyte is 12 nucleotides to 300 nucleotides, more preferred 16 nucleotides to 200 nucleotides or even more preferred 20 nucleotides to 100 nucleotides. The entire lid nucleic acid may have a length of 14 nucleotides to 350 nucleotides, more preferred 18 nucleotides to 250 nucleotides or even more preferred 20 nucleotides to 150 nucleotides.

SEQ ID NO: 9 is an example of a lid nucleic acid that is specific for the *bla*_{OXA-48} gene as an example cfDNA target. Nucleotides 15-88 of SEQ ID NO: 9 are the specific sequence for the *bla*_{OXA-48} gene - they are the reverse complement to SEQ ID NO: 10 which is used in some examples as test target fragment. Nucleotides 1-14 of SEQ ID NO: 9 may be omitted or used to stabilize the strand. Binding of the lid nucleic acid to the nucleic acid nanopore may be facilitated by nucleotides that are also specific for the target, i.e. also the nucleic acid nanopore sequence may be needed to be adapted to a specific target analyte of interest. E.g. SEQ ID NO: 7 and 8 contain sequences for binding a lid nucleic acid. Such sequences may then be also found in the target nucleic acid since the target is again complementary to the lid nucleic acid.

The analyte may be selected from genes of a pathogen, bacterial genes (e.g. bacterial cfDNA), antibiotic resistance genes, cancer-associated genes (in particular genes aberrantly expressed in cancer), and many more. Preferably the analyte is selected from sequences of one of the following genes: NDM, KPC, IMP, VIM, OXA (e.g., OXA-23, OXA-23-like, OXA-48 or OXA-48-like), DHA, CMY, FOX, mecA, mecC, MCR (e.g., mcr-1), vanA, vanB, CTX-M (e.g., CTX-M-2, CTX-M-8, CTX-M 14, or CTX-M 15), mefA, mefE, ermA, ermB, SHV, TEM, FKS, PDR1, ERG11, bla_{OXA-48}, blaKPC, blaVIM, blaNDM. Preferably the analyte is a sequence of an antibiotic resistance gene, including blatem, blast,, blamb, blaoxa, blaZ, aadB, aacC1, aacC2, aacC3, aac6'-lla, aacA4, aad(6'), vanA, vanB, vanC, msrA, sarA, aac(6') aph(2"), vat, vga, ermA, ermB, ermC, int, sul, aac2ia, aac2ib. aac2ic, aac2id, aac2i, aac3ia, aac3iia, aac3iib, aac3iii, aac3iv, aac3ix, aac3vi, aac3viii, aac3vii, aac3x, aac6i, aac6ia, aac6ib, aac6ic, aac6ie, aac6if, aac6ig, aac6iia, aac6iib, aad9, aad9ib, aadd, acra, acrb, adea, adeb, adec, amra, amrb, ant2ia, ant2ib, ant3ia, ant4iia, ant6ia, aph33ia, aph33ib, aph3ia, aph3ib, aph3ic, aph3iiia, aph3iva, aph3va, aph3vb, aph3via, aph3viia, aph4ib, aph6ia, aph6ib, aph6ic, aph6id, arna, baca, bcra, bcrc, bll_acc, bll_ampc, bll_asba, bll_ceps, bll_cmy2, bll_ec, bll_fox, bll_mox, bll_och, bll_pao, bll_pse, bll_sm, b12a_1, b12a_exo, b12a_iii2, bl2a_iii, b12a_kcc, b12a_nps, bl2a_okp, b12a_pc, b12bc_ctxm, bl2be_oxyl, b12be_per, bl2be_shv2, b12b_rob, b12b_teml, b12b_tem2, b12b_tem, b12b_tle, b12b_ula, bl2c_bro, b12c_psel, b12c_pse3, b12d_lcrl, b12d_moxa, bl2d_oxa10, b12d_oxal, bl2d_oxa2, b12d_oxa5, b12d_oxa9, b12d_r39, b12e_cbla, b12e_cepa, b12e_cfxa, b12e_fpm, b12e_y56, b12f_nmca, b12f smel, b12_ges, b12_kpc, b12_1en, b12_veb, b13_ccra, b13_cit, b13_cpha, b13_gim, b13_imp, b13_1, b13_shw, b13_sim, b13_vim, ble, blt, bmr, cara, cata10, catall, cata12, cata13, cata14, cata15, cata16, catal, cata2, cata3, cata4, cata5, cata6, cata7, cata8, cata9, catb 1, catb2, catb3, catb4, catb5, ccoa, ceob, cml_el, cml_e2, cml_c3, cml_e5, eml_e6, cml_e7, cml_e8, dfra10, dfra12, dfra13, dfra14, dfra15, clfra16, dfra17, dfra19, dfral, dfra20, dfra21, dfra22, dfra23, dfra24, dfra25, dfra25, dfra25, dfra26, dfra5, dfra7, dfrbl, dfrb2, dfrb3, dfrb6, emea, emrd, emre, erea, ereb, erma, ermb, ermc, ermd, erme, ermf, ermg, ermh, ermn, ermo, ermq, ermr, erms, ermt, ermu, ermv, ermw, ermx, ermy, fosa, fosb, fosc, fosx, lush, fush, ksga, lmra, Inut, lnua, lnub, lsa, maca, macb, mdtc, mdtf, mdtg, mdth, mdtk, mdtl, mdtm, mdtn, mdto, mdtp, meca, mecrl, mefa, mepa, mexa, mexb, mexc, mexd, mexe, mexf, mexh, mexi, mexw, mexx, mexy, mfpa, mpha, mphb, mphc, msra, norm, olcb, opcm, opra, oprd, oprj, oprm, oprn, otra, otrb, pbpla, pbplb, pbp2b, pbp2, pbp2x, prnra, qac, qaca, qacb, qnra, qnrb, qnrs, rosa, rosb, smea, smeb, smec, smed, smee, smef, srmb, sta, str, sull, sul2, su13, tcma, tcr3, tet30, tet31, tet32, tet33, tet34, tet36, tet37, tet38, tet39, tet40, teta, tetb, tete, tetd, tete, tetg, teth, tetj, tetk, tett, tetm, teto, tetpa, tetpb, tet, tetq, tets, tett, tetu, tetv, tetw, text, tety, tetz, tlrc, tmrb, tole, tsnr, vana, vanb, vane, vand, vane, yang, vanha, vanhb, vanhd, vanra, vanrb, vanrc, vanrd, vanre, vanrg, vansa, vansb, vansc, vansd, vanse, vansg, vant, vante, vantg, vanug, vanwb, vanwg, vanxa, vanxb, vanxd, vanxyc, vanxye, vanxyg, vanya, vanyb, vanyd, vanyg, vanz, vata, vatb, vatc, vatd, vate, vgaa, vgab, vgba, vgbb, vph, ykkc, and ylckd (see the Comprehensive Antibiotic Resistance Database (CARD) available online at card.mcmaster.ca). The lid nucleic acid has preferably a sequence that is complementary to any one of these analytes, especially preferred a complementary sequence of at least 12, at least 16, at least 20 nucleotides in length.

The nucleic acid nanopore can be functionalized with hydrophobic moieties like cholesterol or porphyrin. Such hydrophobic moieties are also referred to as hydrophobic anchors. These moieties/anchors allow an integration of the nucleic acid nanopore into the lipid membrane. The hydrophobic moieties comprised within the embodiments of the present invention are capable of forming non-covalent attractive interactions with lipid bilayers, such as the lipid-based membranes of cells, and act as membrane anchors for the nanopore. According to certain embodiments of the present invention suitable hydrophobic moieties, such as lipid molecules, possessing membrane anchoring properties, may include sterols (including cholesterol, derivatives of cholesterol, phytosterol, ergosterol and bile acid), alkylated phenols (including methylated phenols and tocopherols), flavones (including flavanone containing compounds such as 6-hydroxyflavone), saturated and unsaturated fatty acids (including derivatives such as lauric, oleic, linoleic and palmitic acids), and synthetic lipid molecules (including dodecyl-beta- D-glucoside). The specific hydrophobic moiety anchor may be selected based on the binding performance of the lipid membrane chosen. The hydrophobic moieties can contain a linker or spacer. An example of a linker or spacer is a poly- or oligo-alkyl glycol moiety, such as TEG (triethylene glycol). An example of a hydrophobic moiety with a spacer portion is Cholesterol-TEG. The hydrophobic moiety is preferably attached to the 3' position of a nucleotide, such as in 3' Cholesterol-TEG ("3CholTEG"). In the examples of the invention, SEQ ID NO: 1-6 (or SEQ ID NO: 7 and/or 8 as replacements for SEQ ID NO: 2 and/or 5) have the 3' termini inside the lipid membrane. There, a 3' modification is easily facilitated by chemical derivation. Attaching a hydrophobic moiety to one or more strands of the nanopore is a preferred embodiment of the invention. Cholesterol has been found to be a particularly suitable hydrophobic moiety for use as an anchor in the present invention.

In preferred embodiments for all aspects of the invention, the lipid membrane is a lipid bilayer. Lipid bilayers can be formed by lipids in an aqueous environment on or attached to the solid surface. Preferably the lipids contain phospholipids. Preferably the lipid membrane comprises lipids with a carbon chain with a length of 10 to 24 carbon atoms. Examples of phospholipids include phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, dimyristoyl-1,2-deoxyphosphatidylcholine, plasmalogen, and phosphatidic acid. The lipid membrane may comprise one or a combination of two or more lipids. Fatty acid residues in phospholipids and phospholipid derivatives are not particularly limited, and examples thereof include saturated or unsaturated fatty acid residues having 10 to 24 carbon atoms, preferably 12 to 20 carbon atoms. Specific examples of lipids include lauric acid, myristic acid, or palmitic acid. A preferred lipid is 1,2-diphytanoyl-sn-glycero-3-phosphocholine (DPhyPC). Preferably the lipid membrane has a thickness of 2 nm to 12 nm, more preferred 1 nm to 8 nm. The thickness is determined by the size, e.g**.** chain length, of the chemical moieties constituting the lipid membrane, particularly the lipid molecules. A size can be readily selected to result in the desired thickness. The lipid membrane may further comprise an agent to modify its fluidity, such as sterols (including cholesterol, derivatives of cholesterol, phytosterol, ergosterol and bile acid), alkylated phenols (including methylated phenols and tocopherols), flavones (including flavanone containing compounds such as 6-hydroxyflavone), and unsaturated fatty acids (including derivatives such as lauric, oleic, linoleic and palmitic acids). Such an agent may be in a concentration of at least 8 weight-%, more preferred at least 10 weight-%. Preferably, the lipid membrane, preferably as lipid bilayer, contains cholesterol, in particular preferred at least 8 weight-%, more preferred at least 10 weight-%, cholesterol.

Preferably the lipid membrane is tethered to the solid surface via a hydrophilic chain. Tethering can also or in combination be done by one or more sulphur groups, preferably using a disulfide heterocycle-containing compound such as lipoic acid. Tethering of the lipid membrane to the solid surface can be done by a tethering moiety, also referred to an anchor lipid in case of lipid-containing tethering moieties. The tethering moiety binds both to the lipid membrane and to the solid surface. It may have a hydrophobic portion for binding to the lipid membrane. Preferably it integrates into the lipid membrane. Accordingly, the hydrophobic portion may be the same as a lipid described above for the lipid bilayer. It may further comprise a hydrophilic chain. The hydrophilic chain reaches out of the lipid membrane and creates a distance between the solid surface and the lipid membrane. This distance allows a volume of a medium on the solid surface-facing side of the lipid membrane. From or to said medium a flow through the nanopore may be enabled and potentially measured in the inventive detection method. The tethering moiety may further comprise a moiety for binding to the solid surface. The nature of this moiety depends on the selected solid surface. For metal surfaces, one or more sulphur groups, e.g. a -S-S- group, is/are preferred, e.g. as in a disulfide heterocycle. A preferred example anchor lipid is 2,3-di-O-phytanyl-glycerol-1-tetraethylene glycol-D,L-lipoic acid ester lipid (DPhyTL).

The solid surface may be further bound or covered by a passivating compound, such as mercaptoethanol, to avoid unspecific binding to the solid surface. Such a passivating compound can be used to create distance between the tethering-lipids, and also to increase the volume between the solid surface and the lipid membrane and to increase the fluidity of the lipid membrane, which improves nanopore incorporation efficiency.

The volume between the solid surface and the lipid membrane preferably contains a medium, especially preferred an aqueous medium, especially preferred water and optionally (but preferred), ions, such as metal ions. Preferably the distance between the solid surface and the lipid membrane is 0.5 nm to 10 nm.

The solid surface comprises an electrical conductor, which allows electrical measurements. Example materials for the solid surface are metals. Preferably corrosion resistant metals are used, such as gold, silver, platinum, aluminum or corrosion resistant alloys or combinations thereof. Preferably the solid surface is a metal surface, preferably with a metal comprising gold. Corrosion resistant alloys may comprise iron, copper, nickel, cobalt, chrome, and/or molybdenium, and optionally further carbon.

Preferably the solid surface is a flat surface. Also, preferably the solid surface is smooth with a RMS roughness of at most 10 nm, preferably a RMS roughness of 0.02 nm to 6 nm, preferably of 0.03 nm to 2 nm. The RMS roughness is the quadratic mean, or root mean square (RMS) average of profile height deviations from the mean line. Such height deviations can e.g. be determined by atomic force microscopy. Preferably RMS roughness is as described in Kleinheinz et al., Biosens. Bioelectron. 203: 114024 (2022). An example AFM measurement is conducted using a Cypher ES (Oxford Instruments, United Kingdom) on a vibration isolated table. The measurements were done in 1X PBS under ambient pressure and temperature. In this setup an ARROW-UHFAuD gold-coated silicon cantilever (NanoWorld AG, Switzerland) with resonance frequencies between 700 and 2000 kHz can be used. Operation of the AFM can be conducted through blueDrive technology in tapping mode (TM-AFM) with scan rates between 2 and 8 Hz.

The inventive nanopore assembly may further comprise elements for performing an electric measurement, in particular for detecting an electrical property. The nanopore assembly may comprise a counter electrode and/or a reference electrode. It may also or alternatively comprise a potentiostat or galvanostat. The assembly may also comprise a frequency response analyzer (FRA) in combination or as an alternative to the above. In the inventive method the electrode and/or reference electrode are preferably used on the side of the lipid membrane opposed to/facing away from the solid surface. Such a setup is preferably suited for measuring an electrical property, e.g. selected from impedance, current, voltage or resistance, especially preferred impedance, of the nanopore in the lipid membrane or across the lipid membrane with the nanopore. The surface of the lipid membrane facing away from the solid surface is preferably covered with an aqueous medium, especially preferred a medium comprising water as solvent and optionally (but preferred), ions, such as metal ions. Such a medium on this side of the lipid membrane may further contact the counter electrode and/or reference electrode. An example reference electrode is for example an Ag/AgCl reference electrode. The solid surface may constitute the working electrode.

The invention provides a method of detecting an analyte that binds to a nucleic acid using the inventive nanopore assembly. The method usually comprises providing the nanopore assembly as described herein. The lid nucleic acid is specific to binding an analyte of interest (the analyte). The method comprises contacting the nanopore assembly with a sample comprising the analyte and detecting an electrical property in case the analyte of interest binds the lid nucleic acid. Detecting an electrical property may comprise detecting a difference in an electrical property between a state of the nanopore assembly with and without the analyte, which may in case of analyte detection correlate to a difference between the closed and open state of the nanopore. Preferably the electrical property is impedance, current, voltage or resistance, especially preferred impedance. Preferably electrochemical impedance spectroscopy (EIS) is used. After a detecting step, the lid nucleic acid (which may be displaced by the analyte) can be regenerated. For such a regeneration, the nanopore assembly may be washed to free it of analytes and then contacting it with lid nucleic acids. The lid nucleic acids will then again bind to the nanopore. The measurement is preferably in an aqueous medium, especially preferred a medium comprising water as solvent and optionally (but preferred), ions, such as metal ions. Ions can be used to facilitate detecting or measuring an electrical property.

Further provided is a method of generating the nanopore assembly of the invention. This method comprises immobilizing a lipid membrane to a solid surface, wherein the solid surface comprises or is an electrical conductor and integrating a nucleic acid nanopore into the lipid membrane, wherein the nucleic acid nanopore comprises a lumen with openings, and wherein at at least one of the openings (e.g. at the second opening as mentioned above) the nucleic acid nanopore comprises a lid nucleic acid, wherein said lid nucleic acid comprises a hybridizing sequence that is complementary to a sequence of the nucleic acid nanopore located flanking the opening at which it is located. The nucleic acid nanopore comprises hydrophobic moieties (hydrophobic anchors), e.g. a sterol as mentioned above. These anchors help to allow self-assembly of the nucleic acid nanopore into the lipid membrane. The lipid membrane can be constituted by letting lipids of a vesicle form the membrane on the solid surface. Preferably, first the lipid anchors are bound to the solid surface. Optionally, the solid surface is then passivated, e.g. with mercaptoethanol. Then the lipids are added to combine with the lipid anchors, thus forming the lipid membrane on top of the solid surface. Then the nucleic acids of the nanopore are added, which will self- assemble into the lipid membrane based on the hydrophobic anchor action.

Throughout the present disclosure, the articles "a", "an", and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range while "comprising" still relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

### Examples

The present invention is further illustrated by the following examples, without being limited to these embodiments of the invention.

### Example 1: Materials

Unless otherwise specified, all chemicals were purchased from Sigma-Aldrich. DNA was purchased from Integrated DNA Technologies (IDT). Phospholipids were purchased from Avanti Polar Lipids. 2,3-di-O-phytanyl-glycerol-1-tetraethylene glycol-D,L-lipoic acid ester lipid (DPhyTL) was synthesized by Celestial Synthetics (www.celestialsynthetics.com).

### Example 2: Preparation of template-stripped gold substrates (TSG) and SAM

Gold surfaces were prepared via template stripping, as described previously (Vogel et al. Nanoscale 4: 3820-3832 (2012); Kleinheinz et al., Biosens. Bioelectron. 203: 114024 (2022)). Silicon wafers were sonicated first in acetone, then in pure ethanol and finally in ultrapure water for 10 min, respectively. Subsequently, the wafers were cleaned with acidic piranha solution (3:1 sulfuric acid and 33 % hydrogen peroxide) for 1 hour, and then rinsed thoroughly with ultrapure water and ethanol, followed by drying in a nitrogen stream. A film thickness of 50 nm gold (99.99 % from MaTeck) was deposited by evaporation at a rate of 0.1 Å/s (6 A current, 10⁻⁶ mbar). Standard microscopy slides were then glued to the exposed gold surface using EPO-TEK354 optical adhesive, mixed at a 10:1 ratio of monomer/curing agent and degassed under vacuum at 60 °C for 1 hour before use. The adhesive was deposited at 120 °C for 10 min and cured for 4 hours at 160 °C.

Freshly cleaved TSG slides were rinsed thoroughly with ultrapure ethanol and dried under a stream of nitrogen before being inserted into an ethanol solution of 1 mM DPhyTL and 5 mM mercaptoethanol. Substrates were functionalized at 4 °C overnight and then removed from the solution, rinsed with ultrapure ethanol, dried under a stream of nitrogen, and immediately used for bilayer formation.

### Example 3: Formation of Bilayer

Liposomes (0.8 mg/mL dipalmitoylphosphatidylcholine (DPPC) and 0.2 mg/mL cholesterol in MilliQ) were prepared immediately prior to bilayer formation by extrusion (31x) through track-etched polycarbonate membranes (50 nm pores) using the Avanti mini-extruder kit. Then, 25 µL of vesicle solution was mixed with 25 µL of 1xPBS solution and added immediately to the substrate and incubated at 25 °C overnight. The membrane was then rinsed slowly with 5 mL of 1x PBS solution and monitored for stability via EIS over a period of 2 hours to ensure their electrical properties did not deteriorate before incorporation of the nanopores.

### Example 4: Nanopore folding and incorporation into lipid bilayer

The target-sensing nanopores were designed based on previously described DNA nanopores (Burns et al. Nature nanotechnology 11, 152-156; 10.1038/nnano.2015.279 (2016)). For self-assembly, 1 µM of each strand (1C, 2 dock, 3C, 4C, 5 dock, 6C and Oxa48-lock, see Table 1) were mixed in 100 µL of 0.3 M KCl. Assembly was performed by exposing the mixture to 95 °C for 10 min and then cooling down to 25 °C by 5 °C every 2 min. Ten µL folded nanopores were mixed with 10 µL of 10 mM α-cyclodextrin. The mixture was added on the bilayer and incubated at 25 °C overnight. The next day the membrane was slowly rinsed with 5 mL of 1x PBS solution.

**Table 1.** Names, chemical modifications, and sequences of DNA nucleic acids used to prepare DNA nanopore. Strands 2C and 5C are of conceptional nature and were not used since they lack the sequences for lock strand binding. Strands 2 dock and 5 dock are based on strands 2C and 5C, respectively but have each been extended by an insert (in *italic*)*.* The inserts contain a loop (TTTT) flanked by two lock-binding sequences, which are underlined in strand 2 dock (GGTGACT and CGCAGCC) and bold in strand 5 dock (GTCAAAC and CTGACCG). The corresponding complementary sequences in strand Oxa48-lock are marked likewise underlined (complementary to 2 dock, i.e. AGTCACC and GGCTGCG) or bold (complementary to 5 dock, i.e. GTTTGA and CGGTCAG). These sequences are also found in the strand Oxa48-key, which is complementary to strand Oxa48-lock, except for nt 1-14 of strand Oxa48-lock. The addition "/3CholTEG/" that is added to the sequences of SEQ ID NO: 1, 3, 4, and 6 is an optional 3' Cholesterol-TEG modification. Such a modification can be added or not added at any of the strands. In this example, 4 are added for lipid membrane integration.

| Strand | DNA sequence 5' → 3' |
|---|---|
| 1C | AGCGAACGTGGATTTTGTCCGACATCGGCAAGCTCCCTTTTTCGACT ATT/3CholTEG/   (SEQ ID NO: 1) (50 nt) |
| 2C | CCGATGTCGGACTTTTACACGATCTTCGCCTGCTGGGTTTTGGGAGC TTG   (SEQ ID NO: 2) (50 nt) |
| 2 dock (Oxa-48) | CCGATGTCGGACTTGGTGACTTTTTCGCAGCCTTACACGATCTTCGC CTGCTGGGTTTTGGGAGCTTG (SEQ ID NO: 7) (68 nt) |
| 3C | CGAAGATCGTGTTTTTCCACAGTTGATTGCCCTTCACTTTTCCCAGC AGG/3CholTEG/   (SEQ ID NO: 3) (50 nt) |
| 4C | AATCAACTGTGGTTTTTCTCACTGGTGATTAGAATGCTTTTGTGAAG GGC/3CholTEG/   (SEQ ID NO: 4) (50 nt) |
| 5C | TCACCAGTGAGATTTTTGTCGTACCAGGTGCATGGATTTTTGCATTC TAA   (SEQ ID NO: 5) (50 nt) |
| 5 dock (Oxa-48) | TCACCAGTGAGATTGTCAAACTTTTCTGACCGTTTGTCGTACCAGGT GCATGGATTTTTGCATTCTAA   (SEQ ID NO: 8) (68 nt) |
| 6C | CCTGGTACGACATTTTTCCACGTTCGCTAATAGTCGATTTTATCCAT GCA/3CholTEG/   (SEQ ID NO: 6) (50 nt) |
| Oxa48-lock | CACCGATCGGTGAGCCGAATAATATAGTCACCATTGGCTTCGGTCAG CATGGCTTGTTTGACAATACGCTGGCTGCGCTCCGATACGT (SEQ ID NO: 9) (88 nt) |
| Oxa48-key | ACGTATCGGAGCGCAGCCAGCGTATTGTCAAACAAGCCATGCTGACC GAAGCCAATGGTGACTATATTATTCGG (SEQ ID NO: 10) (74 nt) |

After an analyte measurement, which removes the lock/lid oligonucleotide, the lock/lid can be regenerated. Regeneration of the lock can be achieved by incubation with 5 µl of a 100 µM stock solution of the lock-strand for one hour.

### Example 5: Electrochemical Impedance Spectroscopy (EIS)

EIS measurements were preformed using a dual channel Metrohm Autolab potentiostat with a three-electrode setup using a gold working electrode (gold substrate with the tethered bilayer and nanopore), a platinum wire as a counter electrode and an Ag/AgCl reference electrode in a 3 M KCl buffer solution. The counter electrode was placed at a maximum distance of 1 cm from the working electrode. For each spectrum, a total of 30 frequencies were measured between 100 kHz and 5 mHz with an amplitude of ± 10 mV in 1x PBS buffer. Data were analysed using the ZView4 package (Scribner).

### Example 6: Sample preparation

Genomic DNA of the clinical isolates of *E. coli* and *K*. *pneumoniae* were prepared as described before using the QIAamp DNA Mini Kit (Qiagen) (Conzemius et al. International journal of molecular sciences 22; 10.3390/ijms222413654 (2021)). In some experiments, the DNA analyte was fragmented with a M220 focused ultrasonicator (Covaris) with the parameters: power 75 W, duty factor 10 %, cycles per burst 200 and 3 x 60 sec. Directly before applying the gDNA on the nanopore, it was incubated at 95 °C for 10 min and placed afterwards immediately on ice. This procedure increases the amount of single-stranded DNAs.

### Example 7: Results and Discussion

As mentioned in the background section, there is a need to improve DNA sensing methods. To improve the use of oligonucleotide analytics, a sequence-selective DNA nanopore was created and used to detect as analyte the clinically relevant antibiotic resistance (ABR) gene *bla*_{OXA-48} encoded on short DNA strands and on bacterial DNA extracts, respectively as a showcase of the method. The analyte gene triggered the opening and closing of a sequence-specific DNA nanopore (Figure 1). The advantages of nanopore-based detection principles are that i) no labelling is required, ii) single molecule detection is feasible, iii) reusability enables multiple uses, iv) real-time analysis without the need of amplification is possible, and v) high target specificities can be achieved. These features make the inventive nanopore an optimal platform for rapid diagnostic tests. DNA nanopores offer many possibilities regarding their rational design and the integration of programmable nucleic acids interactions.

A very simple six-helix-bundle DNA nanopore was used, which is, in contrast to protein-based nanopores, very easy to produce due to the DNA-driven self-assembly process and the short length (with nucleic acids of 50 base pairs (bp), 68 bp, and 88 bp) of the six individual building blocks (SEQ ID NOs: 1-6, with the additional lid nucleic acid of SEQ ID NO: 9 and with further modifications in two strands to include a lid-binding loop in each of SEQ ID NOs 2 and 5 as shown in sequences of SEQ ID NOs 7 and 8, respectively). This DNA nanopore is used for the label-free detection of short DNA strands, such as cfDNA (Figure 2). To target a cfDNA in diagnostic applications, the lid nucleic acid was designed to target the *bla*_{OXA-48} gene as an example cfDNA. To evaluate the sensing capacity of the DNA nanopore, we first added different concentrations of single-stranded DNA oligonucleotides corresponding to the *bla*_{OXA-48} gene to the nanopore sensor and performed impedance measurements to track the opening of the pore (Figure 3). In the closed state, the nanopore impedes ions to cross the membrane, resulting in a high membrane impedance of approximately 1 MΩ.cm⁻². When the lid/lock-strand is removed, ions can cross the lipid membrane during impedance measurements, resulting in a reduction of membrane resistance of up to 80% with increasing key-concentrations.

The sensor response correlated with the concentration of *bla*_{OXA-48} oligonucleotides indicating that at least semi-quantitative measurements are feasible with the nanopore. In a single molecule detection setup, this technology could pave the way for the bias-free quantification of nucleic acids since no amplification reaction is required. The addition of non-target control oligonucleotides revealed that the lid was only removed in the presence of a correct target sequence as analyte. The encouraging specificity results, enable applications detecting single nucleotide polymorphisms (SNPs).

The nanopore is combined with a lipid bilayer on a solid surface. Phospholipid bilayers have evolved to be highly resistant to nonspecific binding events, which significantly enhances the selectivity and signal to noise ratio of charge transport through the embedded nanopores (Lee et al., Nature Communications 12:3741 (2021)). In combination with the high selectivity of the lock-strand (lid nucleic acid) for the target sequence (analyte), the non-specific signal generated by the sensor could be significantly reduced. The measurements shown in Fig. 3 were carried out on lipid bilayers with a surface area of 0.28 cm², where a limit of detection of 10 pM was possible. Sensor response can be significantly improved by reducing the area of the lipid membrane. This reduces the number of defects causing background ion leakage that obscures nanopore opening events. The lipid bilayer can e.g. be as described in Kleinheinz et al., ACS Appl. Mater. Interfaces 15: 46655-46667 (2023).

To evaluate our nanosensor under more realistic conditions, we selected four clinical isolates of *K. pneumoniae* and *E. coli* each with and without the *bla*_{OXA-48} gene, respectively. Optionally, the DNA can be randomly fragmented using ultrasonication. A main question was how the nanopore will behave in the presence of large amounts of non-target DNA fragments and if these could trigger unintended strand displacement reactions. Analogous to the single-stranded DNA experiments, the nanopore sensor responded highly specifically upon addition of bacterial genomic DNA (gDNA) to the presence of the *bla*_{OXA-48} gene independent of the bacterial species (Fig. 3 B & C). DNA extracts of isolates lacking the *bla*_{OXA-48} gene did not generate significant sensor responses. The impedance changed depending on the input gDNA concentrations confirming that the DNA nanopore could be used as quantitative sensor. Furthermore, we were able to regenerate the pore by addition of a new lid nucleic acid, after the lid has been previously removed by a target addition, and conduct a second follow-up measurement (Fig. 3C, column "Lock").

Finally, it was investigated if the DNA nanopore sensor can detect also short gDNA fragments. To simulate microbial cfDNA, the bacterial gDNA was randomly fragmented using ultrasonication. As expected, the sensor response was weaker most probably due to the fragmentation of the target sequences (Figure 3). Nevertheless, the presence of the *bla*_{OXA-48} gene could be confirmed indicating that the sensor may be used for the analysis of cfDNA.

In conclusion, we present a label-free and amplification-independent sensing concept based on a sequence-selective DNA nanopore. The system is at least semi-quantitative and can be regenerated for multiple uses.

## Claims

1. A nanopore assembly comprising a nucleic acid nanopore that penetrates a lipid membrane, wherein said lipid membrane is tethered to a solid surface, and with the nucleic acid nanopore comprising a lumen with openings, with a first openings oriented at the solid surface and a second opening oriented away from the surface, wherein at at least one of the openings the nucleic acid nanopore comprises a lid nucleic acid, wherein said lid nucleic acid comprises a hybridizing sequence that is complementary to a sequence of the nucleic acid nanopore located flanking the opening at which it is located, and wherein the solid surface comprises an electrical conductor.

2. The nanopore assembly of claim 1, wherein the lid nucleic acid is across the second opening.

3. The nanopore assembly of claim 1 or 2, further comprising counter electrode and/or reference electrode; and/or further comprising a potentiostat or galvanostat.

4. The nanopore assembly of any one of claims 1 to 3, wherein the nucleic acid nanopore comprises nucleotides of which 80% to 100% are DNA nucleotides.

5. The nanopore assembly of any one of claims 1 to 4, wherein the nucleic acid nanopore comprises at least 3 nucleic acid double helices, preferably the nucleic acid nanopore comprises 4 to 25 nucleic acid double helices.

6. The nanopore assembly of any one of claims 1 to 5, wherein the nucleic acid nanopore comprises nucleic acid double helices with a length of 20 base pairs to 250 base pairs, preferably 22 base pairs to 100 base pairs.

7. The nanopore assembly of any one of claims 1 to 6, wherein the lumen has a diameter of at least 1.2 nm along its length from first opening to second opening.

8. The nanopore assembly of any one of claims 1 to 7, wherein the solid surface is a metal surface, preferably with a metal comprising gold.

9. The nanopore assembly of any one of claims 1 to 8, wherein the solid surface is a flat surface, preferably with a RMS roughness of at most 10 nm, preferably a RMS roughness of 0.02 nm to 6 nm.

10. The nanopore assembly of any one of claims 1 to 9, wherein the lipid membrane comprises lipids with a carbon chain with a length of 10 to 24 carbon atoms.

11. The nanopore assembly of any one of claims 1 to 10, wherein the lipid membrane is tethered to the solid surface via a hydrophilic chain and/or by one or more sulphur group.

12. The nanopore assembly of any one of claims 1 to 11, wherein the distance between the solid surface and the lipid membrane is 0.5 nm to 10 nm and/or the lipid membrane has a thickness of 1 nm to 8 nm and/or the nucleic acid nanopore has a length of 4 to 30 nm.

13. A method of detecting an analyte that binds to a nucleic acid, comprising providing a nanopore assembly of any one of claims 1 to 12, wherein the lid nucleic acid is specific to binding an analyte of interest, comprising contacting the nanopore assembly with a sample comprising the analyte and detecting an electrical property in case the analyte of interest binds the lid nucleic acid.

14. The method of claim 13, wherein the electrical property is impedance.

15. Method of generating the nanopore assembly of any one of claims 1 to 12, comprising immobilizing a lipid membrane to a solid surface, wherein the solid surface comprises an electrical conductor and integrating a nucleic acid nanopore into the lipid membrane, wherein the nucleic acid nanopore comprises a lumen with openings, and wherein at at least one of the openings the nucleic acid nanopore comprises a lid nucleic acid, wherein said lid nucleic acid comprises a hybridizing sequence that is complementary to a sequence of the nucleic acid nanopore located flanking the opening at which it is located, and further comprising hydrophobic anchors.
